# FASCICULE DE BREVET EUROPEEN

(11) **EP 2 334 256 B1**
(45) Date de publication et mention de la délivrance du brevet: **25.04.2012**
(21) Numéro de dépôt: 09748127.9
(22) Date de dépôt: 17.09.2009
(51) Int. Cl.: A61F 2/00

(54) **INSTRUMENT CHIRURGICAL POUR DÉPLOYER UNE PROTHÈSE**
OPERATIONSINSTRUMENT ZUR ABLAGE EINER PROTHESE
SURGICAL INSTRUMENT FOR DEPLOYING A PROSTHESIS

(30) Priorité: 18.09.2008 US 97986 P
(43) Date de publication de la demande: 22.06.2011
(73) Titulaire: Sofradim Production, 01600 Trevoux (FR)
(72) Inventeur: BAILLY, Pierre, F-69300 Caluire (FR); DOUCET, Geneviève, F-69400 Villefranche Sur Saone (FR)
(74) Mandataire: Maureau, Philippe
(86) Numéro de dépôt international: PCT/FR2009/051749
(87) Numéro de publication internationale: WO 2010/031967

(56) Documents cités:
- EP-A- 1 336 391
- FR-A- 2 208 299
- US-A- 5 258 000

## Description

La présente invention a pour objet un instrument chirurgical permettant la pose d'une prothèse destinée, en particulier à l'obturation des hernies, ainsi qu'un kit comprenant cet instrument chirurgical et cette prothèse.

La paroi abdominale chez l'être humain est constituée de graisses et de muscles reliés entre eux par des aponévroses. Il arrive qu'au niveau des aponévroses se forme une rupture de continuité laissant passer une partie du péritoine qui constitue alors un sac, ou encore une hernie, contenant soit de la graisse soit une partie des intestins. Les hernies ou éventrations (hernie survenant sur une cicatrice pariétale chirurgicale) se manifestent par une excroissance à la surface de la peau et sont qualifiées de hernies ou éventrations, par exemple, ombilicales ou inguinales en fonction de leurs localisations.

La méthode la plus classique pour réparer un défaut herniaire fait intervenir la pose de fils de sutures sous tension. Cependant, ce type de réparations est source de douleurs pour le patient et, du fait des tensions importantes, présente un risque non négligeable de déchirure des muscles et aponévroses par les sutures et/ou de récidive de la hernie.

Afin de minimiser les risques de récidive, les chirurgiens ont fréquemment recours à la mise en place d'une prothèse en treillis synthétique qui remplace ou renforce les tissus anatomiques affaiblis sans nécessiter le rapprochement des bords des tissus lésés. Toutefois, une telle prothèse est soumise à une pression abdominale qui tend à l'expulser vers l'extérieur. Or, l'efficacité de la prothèse, et donc la minimisation des risques de rechute, dépendent en grande partie de la fixation de celle-ci. Tout d'abord, l'étalement des prothèses, qui sont souvent souples, s'avère difficile de sorte qu'elles ont tendance à former des plis sur la paroi abdominale. L'absence d'étalement parfait entraîne un risque d'engagement du sac péritonéal et augmente les possibilités de récidive. Il est donc essentiel pour le chirurgien de s'assurer qu'aucune partie de la prothèse n'est repliée et qu'aucun viscère ou qu'aucune partie des intestins ne s'interpose entre la prothèse et la paroi abdominale. Ensuite, un mauvais positionnement des sutures ou une mauvaise fixation de la prothèse risque de distordre celle-ci et de créer des tensions.

Le document D1 (FR 2 208 299) décrit une prothèse pour le traitement des hernies inguinales et hiatales comprenant une plaque munie d'un orifice et de crochets.

Le document D2 (EP 1 336 391) décrit une prothèse comprenant deux couches souples définissant une poche, au sein de laquelle un fil ressort peut être introduit pour réaliser le déploiement de la prothèse.

La présente invention a pour but de proposer un instrument chirurgical permettant de faciliter l'étalement et la fixation d'une prothèse utilisable pour le traitement chirurgical des hernies et permettant de remédier aux inconvénients précités, en particulier, mais pas seulement, pour le traitement chirurgical des hernies de petites tailles.

La présente invention concerne également un kit chirurgical pour le traitement d'un défaut herniaire de la paroi abdominale suivant les revendications 1 à 8.

Dans la présente demande, on entend par prothèse un dispositif médical biocompatible implantable dans le corps humain, ou animal.

Un premier élément du kit comporte un instrument chirurgical pour le déploiement d'une prothèse destinée à combler un défaut herniaire de la paroi abdominale, ladite prothèse comprenant au moins une première couche en matériau souple biocompatible destinée à être placée en regard de la paroi abdominale et au moins une deuxième couche en matériau souple biocompatible destinée à être placée en regard de la cavité abdominale, lesdites première et deuxième couches étant assemblées entre elles de façon à définir un espace interne accessible audit instrument chirurgical au moyen d'un orifice ménagé dans la dite première couche, ledit instrument chirurgical comprenant au moins une feuille en matériau souple et élastique, ladite feuille se superposant sur elle-même une ou plusieurs fois de façon continue afin de définir plusieurs niveaux (ou couches) formant une spirale.

Du fait de l'élasticité de ladite feuille, la spirale est apte à adopter une configuration substantiellement plate, dans laquelle chaque niveau est en contact avec le niveau adjacent, correspondant à une configuration au repos dans laquelle la spirale ne subit pas de contraintes significatives, ou au contraire une configuration déployée, dans laquelle aucun niveau n'est en contact avec un autre niveau, correspondant à une configuration dans laquelle une force tendant à éloigner l'une de l'autre les deux extrémités de la spirale est exercée. Dans la présente demande, on se référera à l'instrument chirurgical faisant partie du kit selon l'invention également par les termes disque de protection, ou encore disque, ou encore spirale.

Grâce à l'élasticité du matériau constituant ladite feuille, le disque ou spirale présente un effet ressort : ainsi si, dans sa configuration plate, on le plie en le pressant sur lui-même en exerçant une pression sur ses bords, il tend à revenir naturellement dans sa configuration plate lorsqu'on relâche la pression exercée.

Comme il apparaîtra de la description qui suit, l'instrument chirurgical faisant partie du kit selon l'invention est destiné à être introduit dans la prothèse au sein de l'espace interne de cette dernière, afin de faciliter l'introduction, la mise en place et le déploiement de la prothèse dans le site d'implantation. L'instrument chirurgical doit ensuite être retiré de la prothèse une fois celle-ci fixée au site d'implantation.

Dans une forme de réalisation de l'invention, l'extrémité supérieure de la spirale (ou disque) comprend une languette d'extraction. Ainsi, une fois que la prothèse est correctement déployée et positionnée, puis fixée, le chirurgien peut retirer l'instrument chirurgical ou disque de la prothèse en tirant sur la languette d'extraction : il déploie alors le disque qui s'étend sous forme de spirale et le chirurgien peut alors retirer de façon aisée le disque en le faisant tourner sur lui-même ou plus facilement en exerçant une traction linéaire simple vers le haut sur la languette : compte tenu de sa forme et de son matériau, ayant de préférence un faible coefficient de frottement vis-à-vis de la prothèse, la spirale se déroule automatiquement.

Dans une forme de réalisation de l'invention, le matériau constituant ladite feuille est un polymère choisi parmi le polypropylène, le polyéthylène, le polytétrafluoréthylène (PTFE), et/ou leurs mélanges, par exemple possédant un faible coefficient de frottement avec la prothèse pour faciliter son extraction.

La présente invention a pour objet, suivant la revendication 1, un kit chirurgical pour le traitement d'un défaut herniaire de la paroi abdominale comprenant :
- un instrument chirurgical tel que décrit ci-dessus ;
- et une prothèse destinée à combler ledit défaut, ladite prothèse comprenant au moins une première couche en matériau souple biocompatible destinée à être placée en regard de la paroi abdominale et au moins une deuxième couche en matériau souple biocompatible destinée à être placée en regard de la cavité abdominale, lesdites première et deuxième couches étant assemblées entre elles de façon à définir un espace interne ouvert au moyen d'un orifice ménagé dans ladite première couche.

Dans une forme de réalisation, le kit chirurgical selon l'invention comporte une pluralité de fils de centrage destinés à être liés à ladite prothèse sur le pourtour dudit orifice. Il est entendu que les fils de centrage peuvent être préalablement fixés à ladite prothèse ou encore que lesdits fils de centrage pourront être fixés ultérieurement par le chirurgien et/ou enlevés en fin d'intervention.

Dans une forme de réalisation du kit chirurgical, au moins une des couches de ladite prothèse est constituée d'un arrangement de fils. De façon préférentielle, les deux couches sont constituées d'un arrangement de fils.

Dans une forme de réalisation de l'invention, la deuxième couche de ladite prothèse du kit est recouverte d'un revêtement anti-adhérent sur sa face destinée à être mise en regard de la cavité abdominale.

Par « anti-adhérent », on entend au sens de la présente demande un matériau ou un revêtement biocompatible lisse et non poreux n'offrant pas d'espace à une recolonisation cellulaire.

Le revêtement anti-adhérent selon la présente invention permet de protéger au moins pendant la phase initiale de cicatrisation la seconde couche de ladite prothèse, à savoir qu'elle n'est pas exposée aux cellules inflammatoires, comme les granulocytes, les monocytes, les macrophages ou encore les cellules géantes multinucléées généralement activées par le geste chirurgical. En effet, au moins durant la phase initiale de cicatrisation, dont la durée peut varier de 5 à 10 jours environ, seul le revêtement anti-adhérent est accessible par les différents facteurs tels que les protéines, les enzymes, les cytokines ou les cellules de la lignée inflammatoire, au niveau de la première partie de textile.

Dans le cas où le revêtement anti-adhérent est constitué de matériaux non résorbables, il protège ainsi la couche de prothèse enduite avant et après implantation, sur toute la durée d'implantation de la prothèse.

Par ailleurs, grâce au revêtement anti-adhérent, les tissus fragiles environnants tels que les viscères creux, par exemple, sont protégés, en particulier de la formation d'adhérences fibreuses sévères post-chirurgicales non souhaitées.

Dans le cas où le matériau anti-adhérent comprend un matériau biorésorbable, il est préférable de choisir un matériau biorésorbable qui ne se résorbe pas avant plusieurs jours afin que le revêtement anti-adhérent puisse assurer sa fonction de protection de l'intestin et des organes creux durant les jours suivant l'opération, et, jusqu'à ce que la réhabilitation cellulaire de la prothèse protège à son tour les organes fragiles.

D'autres caractéristiques et avantages de l'invention apparaîtront au cours de la description qui va suivre d'un mode particulier de réalisation, donné uniquement à titre d'exemple non limitatif, dans lequel la prothèse est un renfort de paroi abdominale.
- la Figure 1 est une représentation en coupe d'une hernie ou éventration médiane abdominale ;
- la Figure 2 est une représentation en perspective d'une prothèse contenue dans le kit selon la présente invention ;
- la Figure 3 est une représentation en coupe de la prothèse de la Figure 2 ;
- la Figure 4 est une représentation schématique en coupe de la paroi abdominale montrant une réparation d'un défaut herniaire qui a été effectuée à l'aide d'un kit chirurgical selon la présente invention ;
- la figure 5 est une vue en perspective d'une forme de réalisation de l'instrument chirurgical contenu dans le kit selon l'invention, dans sa configuration plate ;
- la Figure 6 est une vue en perspective de l'instrument chirurgical de la figure 5, dans sa configuration déployée ;
- la Figure 7 est une vue en perspective d'une forme de réalisation du kit selon l'invention, l'instrument chirurgical étant totalement introduit dans l'espace interne de la prothèse ;
- la Figure 8 est une représentation schématique en coupe de la paroi abdominale montrant l'introduction dans le site d'implantation du kit de la figure 7,
- la Figure 9 est une vue en perspective du kit de la figure 7, une fois redéployé dans le site d'implantation (ce dernier n'étant pas représenté) ;
- la Figure 10 est une vue en perspective de l'étape d'extraction de l'instrument chirurgical de la prothèse, une fois cette dernière fixée dans le site d'implantation (ce dernier n'étant pas représenté).

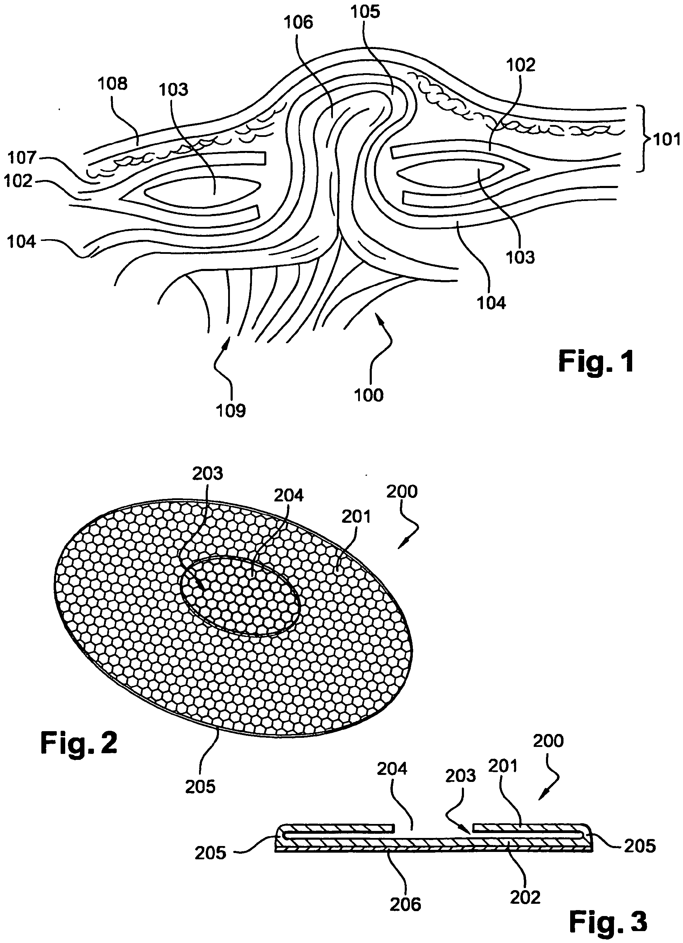

La figure 1 représente un défaut herniaire 100 de la paroi abdominale 101 qui se caractérise par une rupture de continuité de l'aponévrose 102 entourant les muscles droits 103 et un passage du péritoine 104 formant un sac, le sac herniaire 105, qui contient soit de la graisse (épiploon) soit une partie des viscères 106, et qui fait alors pression sur les tissus graisseux 107 et affleure la peau 108. Un traitement d'un défaut herniaire 100 consiste à replacer et maintenir les viscères 106 dans la cavité abdominale 109.

Les Figures 2 et 3 représentent une prothèse 200 apte à combler un défaut herniaire 100 tel que celui représenté à la figure 1, ladite prothèse 200 comprenant au moins une première couche 201 en matériau souple biocompatible destinée à être placée en regard de la paroi abdominale 101 et au moins une deuxième couche 202 en matériau biocompatible destinée à être placée en regard de la cavité abdominale 109. Lesdites première et deuxième couches (201, 202) sont assemblées entre elles de façon à définir un espace interne 203 ouvert par un orifice 204 ménagé dans ladite première couche 201 et s'étendant jusqu'à la zone d'assemblage desdites première et deuxième couches 201, 202.

Dans l'exemple représenté, la première couche 201 et la deuxième couche 202 sont constituées d'arrangements de fils, tels que des tissus, non-tissés ou des tricots, et elles sont assemblées sur leur périphérie par une couture 205. Les fils formant les couches (201, 202) peuvent être choisis parmi des fils résorbables et/ou non résorbables. Dans l'exemple représenté, un revêtement anti-adhérent 206, de préférence biorésorbable, recouvre avantageusement la surface externe de la deuxième couche 202 afin d'éviter en particulier la formation d'adhérences fibreuses sévères post-chirurgicales non souhaitées.

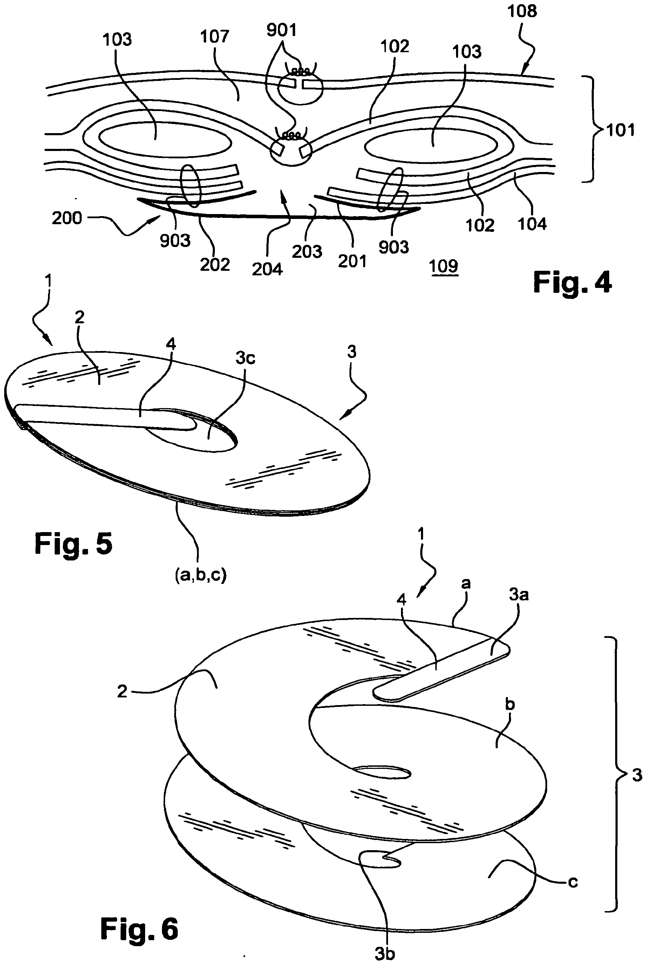

La prothèse 200 des figures 2 et 3 est destinée à la réparation d'un défaut herniaire 100 tel que celui de la figure 1, et doit être disposée, après implantation, comme montré sur la figure 4. Sur cette figure, la prothèse 200 est schématisée, après réduction du sac herniaire 105, telle que mise en place pour combler le défaut herniaire 100 : comme il apparaît de cette figure, sur laquelle sont schématisées des sutures (901 ; 903), le chirurgien a incisé la peau 108 et l'aponévrose 102 pour introduire la prothèse 200 dans le défaut herniaire ; il a ensuite disposé la prothèse 200, avec sa première couche 201 en regard de la paroi abdominale 101 et sa deuxième couche 202 en regard de la cavité abdominale 109 ; il a fixé la prothèse 200 en suturant la couche 201 à l'aponévrose 102 et au péritoine 104 à l'aide des sutures 903 puis il a refermé les incisions initiales de l'aponévrose 102 et de la peau 108 à l'aide des sutures 901.

Lors d'une telle opération, la difficulté réside dans l'introduction et le déploiement de la prothèse 200, en particulier son étalement et son placage contre la paroi abdominale 101, alors que par ailleurs les incisions initiales de la peau 108 et de l'aponévrose 102 devant être les plus petites possibles, l'espace de travail et la visibilité du chirurgien sont particulièrement limités.

Les figures 5 et 6 représentent une forme de réalisation d'un instrument chirurgical 1 faisant partie du kit selon l'invention, ledit instrument 1 étant particulièrement utile pour l'introduction et le déploiement de la prothèse 200 lors de l'opération décrite ci-dessus.

Comme il apparaît des figures 5 et 6, l'instrument chirurgical 1 faisant partie du kit selon l'invention comprend au moins une feuille 2 en matériau souple et élastique, ladite feuille 2 se superposant sur elle-même une ou plusieurs fois de façon continue afin de définir plusieurs couches ou niveaux (a, b, c) formant une spirale 3. Comme il apparaît de la figure 6, la spirale 3 est finie (ou bornée) et possède deux extrémités opposées, une extrémité supérieure 3a et une extrémité inférieure 3b. Du fait de l'élasticité de ladite feuille 2, la spirale 3 peut adopter une configuration substantiellement plate, dans laquelle chaque niveau (a, b, c) est en contact avec le niveau (a, b, c) adjacent, correspondant à une configuration au repos dans laquelle la spirale 3 ne subit pas de contraintes significatives : cette configuration est montrée sur la figure 5. Alternativement, la spirale 3 peut adopter une configuration déployée, dans laquelle aucun niveau (a, b, c) n'est en contact avec un autre niveau, correspondant à une configuration dans laquelle une force, par exemple exercée par le chirurgien, tendant à éloigner l'une de l'autre les deux extrémités (3a, 3b) de la spirale 3 est exercée : cette configuration est montrée sur la figure 6. L'instrument chirurgical est ainsi capable d'adopter deux configurations : une configuration plate, telle que montrée sur la figure 5, et une configuration déployée, telle que montrée sur la figure 6.

Comme il apparaît de la figure 5, dans sa configuration plate, la spirale 3, du fait de la faible épaisseur de la feuille 2, forme un disque plat, muni d'un trou central 3c.

Le matériau constituant ladite feuille 2 est de préférence un polymère type polypropylène, polyéthylène, ou polytétrafluoréthylène (PTFE) ; un tel polymère permet de donner à la feuille l'élasticité nécessaire pour passer de sa configuration de spirale plate à celle de spirale déployée. Par ailleurs, comme il apparaîtra de la suite de la description, le matériau constituant ladite feuille 2, permet à l'instrument chirurgical 1, lorsqu'il est dans sa configuration de spirale plate montrée sur la figure 5, d'être replié en deux sur lui-même sous l'effet d'une pression exercée sur deux bords opposés, par exemple selon un pli correspondant à un de ses diamètres : dans un tel cas, du fait de l'élasticité de la feuille 2, l'instrument chirurgical 1 tend à revenir naturellement dans sa configuration plate lorsqu'on relâche la pression exercée.

Le disque ou instrument chirurgical 1 possède ainsi des propriétés d'élasticité (redéploiement après pliage), de rigidité (maintien de la prothèse contre la paroi abdominale), de souplesse (facilitant son extraction) : l'instrument chirurgical 1 faisant partie du kit selon l'invention joue également un rôle de protection, comme il apparaîtra plus loin, dans la zone de la couture 205 de la prothèse, lors de la fixation de cette dernière à la paroi abdominale, contre des perforations éventuelles par les aiguilles de suture ou les attaches de l'agrafeuse.

Comme il apparaît de la figure 6, l'extrémité supérieure 3a de la spirale (ou disque) 3 comprend une languette d'extraction 4.

L'instrument chirurgical 1 peut être introduit extrêmement aisément dans une prothèse 200 telle que représentée à la figure 2 : en effet, pour ce faire, il suffit, en utilisant la spirale 3 de la feuille 2 sous sa forme déployée telle que montrée à la figure 6, d'introduire l'extrémité inférieure 3b de la spirale 3 dans l'orifice 204 de la prothèse 200 puis de faire tourner la spirale 3 par rapport à la prothèse 200 en faisant coulisser la feuille 2 dans l'espace interne 203 de la prothèse 200.

De préférence, le matériau constituant la feuille 2 possède un faible coefficient de frottement vis-à-vis de la prothèse 200 pour faciliter à la fois son introduction dans la prothèse 200, et comme on le verra plus loin son extraction. Par exemple, si les fils constituant la prothèse sont en polyester, ou en polypropylène, et la feuille 2 est en polypropylène, ou en polytétrafluoroéthylène, la feuille 2 glissera aisément contre la prothèse 200 car le polyester et le polypropylène possèdent un faible coefficient de frottement vis-à-vis du polypropylène et vis-à-vis du polytétrafluoroéthylène.

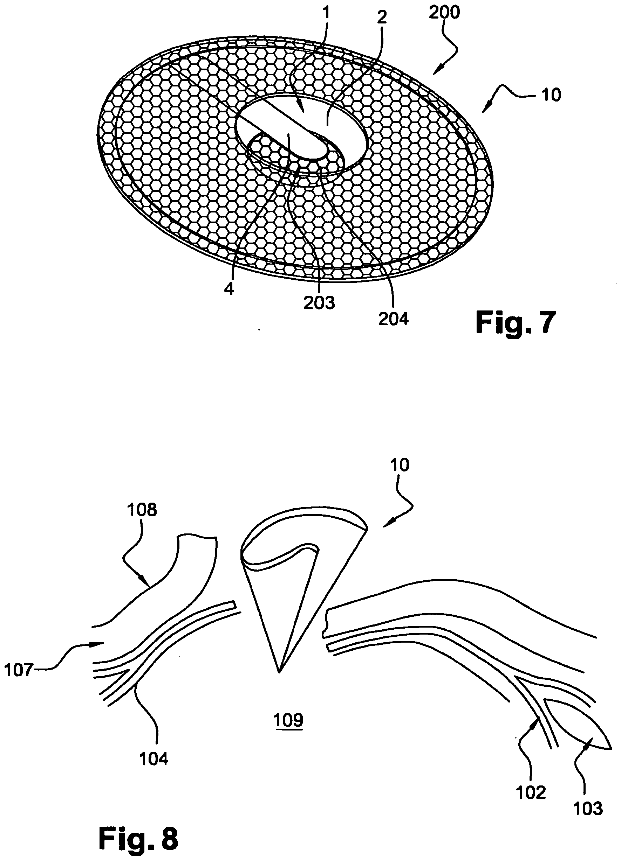

De cette façon, la feuille 2 peut être introduite complètement au sein de l'espace interne 203 de la prothèse 200, comme montré sur la figure 7. Comme il apparaît de cette figure, une fois que l'instrument chirurgical 1 est totalement introduit dans la prothèse 200, la spirale 3 est replacée dans sa configuration plate (correspondant à la figure 5), avec la languette d'extraction 4, située à l'extrémité supérieure 3a de la spirale 3, visible par l'orifice 204.

Le kit 10 ainsi constitué de la prothèse 200 et de l'instrument chirurgical 1 totalement introduit au sein de l'espace ouvert 203 de la prothèse 200, est totalement plat. Du fait de l'élasticité de la feuille 2 formant l'instrument chirurgical 1 et de la souplesse naturelle de la prothèse 200, dont les couches (201, 202) sont des arrangements de fils, le chirurgien peut saisir le kit 10 (prothèse 200 + instrument 1) et le plier en deux, voire en 4, comme montré sur la figure 8, en exerçant une pression sur les bords du kit 10 afin de l'introduire dans le site d'implantation dans la cavité abdominale 109 par les incisions pratiquées au niveau de la peau 108 et de l'aponévrose 102. Du fait qu'il est replié sur lui-même, le kit 10 occupe peu de volume et est aisément introduit dans ces incisions de tailles limitées.

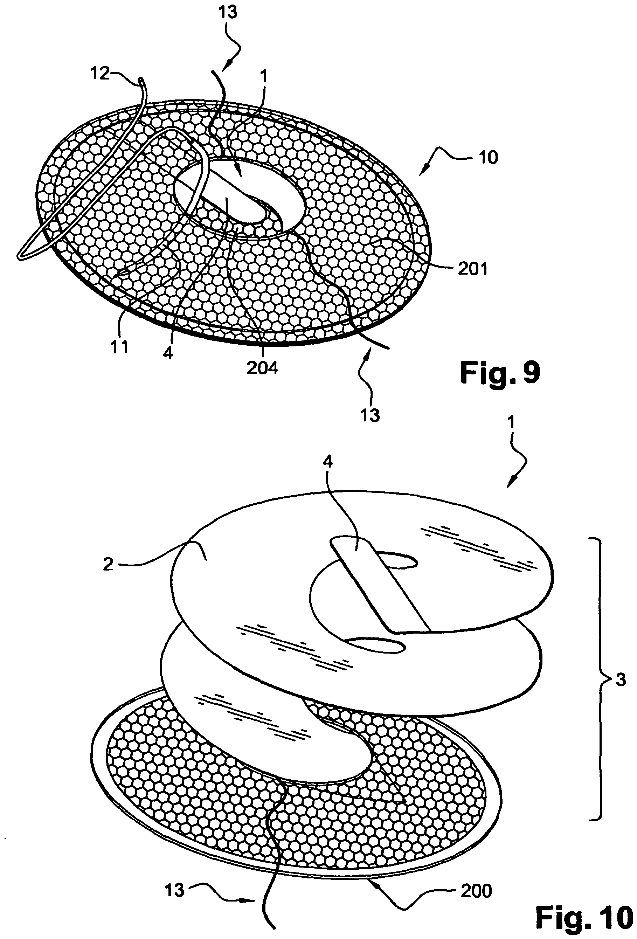

Une fois dans la cavité abdominale 109, le kit 10 se redéploie naturellement, grâce à l'élasticité de la feuille 2, qui tend à revenir naturellement dans sa position de spirale 3 plate (correspondant à la figure 5). Sur la figure 9, est représenté le kit 10, tel que redéployé dans la cavité abdominale : sur cette figure toutefois, pour des raisons de clarté, le site d'implantation n'a pas été représenté à nouveau. Grâce à la présence de l'instrument chirurgical 1, à sa forme et à sa structure, et en particulier à ses propriétés élastiques, le chirurgien sait alors que la prothèse 200 est totalement déployée, et que sa couche 201 en particulier est avantageusement plaquée contre l'aponévrose 102 de la paroi abdominale, sans former de plis risquant d'entraîner des sutures malencontreuses avec des organes fragiles environnants comme les viscères. Le chirurgien peut alors suturer la couche 201 de la prothèse 200 à l'aponévrose 102 de la paroi abdominale, à l'aide d'une aiguille 11 et d'un fil 12 par exemple, comme montré sur la figure 9. Il exécute ainsi les sutures 903 représentées à la figure 4, à la périphérie de la prothèse 200, formant ainsi des moyens d'attaches de la prothèse 200. Alternativement, le chirurgien pourrait utiliser une agrafeuse, par exemple une agrafeuse de coelioscopie, et des agrafes comme moyens d'attaches. Lors de cette opération, l'instrument chirurgical 1 faisant partie du kit selon l'invention joue un rôle de protection des viscères environnants, en particulier au voisinage de la zone de la couture 205 de la prothèse 200, en évitant, du fait de la présence de la feuille 2 qui forme barrière, des perforations éventuelles de ces viscères par les aiguilles de suture ou les attaches de l'agrafeuse.

Au cours de cette opération, le chirurgien peut s'aider, pour centrer le kit 10, et donc la prothèse 200, sur le défaut à combler, de fils de centrage 13, déjà liés; ou qu'il aura préalablement liés, à la prothèse 200 sur le pourtour de l'orifice 204 de cette dernière, comme montré sur la figure 9. Ces fils de centrage 13 sont de préférence retirés une fois la prothèse 200 fixée.

Une fois la prothèse 200 ainsi fixée, le chirurgien peut retirer l'instrument chirurgical 1, en l'extrayant de la prothèse 200 par l'orifice 204 : pour ce faire, il tire sur la feuille 2 à l'aide de la languette d'extraction 4 qu'il saisit aisément par l'orifice 204, et il déploie la spirale 3 tout en la faisant tourner vis-à-vis de la prothèse 200, comme montré sur la figure 10. L'instrument chirurgical 1 est ainsi aisément extrait de la prothèse 200, en particulier lorsque le coefficient de frottement du matériau constituant la feuille 2 est faible par rapport à la prothèse 200.

Il ne reste alors au chirurgien qu'à refermer les incisions initiales de l'aponévrose 102 et de la peau 108 par les sutures 901 telles que montrées à la figure 4.

L'instrument chirurgical 1 faisant partie du kit selon l'invention permet ainsi un déploiement et un étalement de la prothèse 200 les plus efficaces possibles lors d'une opération de réduction d'un défaut herniaire. En particulier, du fait de son effet ressort et par son aptitude à rigidifier la prothèse, l'instrument chirurgical faisant partie du kit selon l'invention permet à la fois de réduire le volume occupé par la prothèse lors de son introduction dans le site d'implantation, et de réaliser un placage optimal de cette dernière contre la paroi abdominale, permettant ainsi d'éviter la formation de plis non souhaités au sein de la prothèse.

Une méthode de traitement ou de prévention d'un défaut herniaire dans la région ombilicale (ne faisant pas partie de l'invention), en disposant d'une prothèse et d'un instrument chirurgical (ou disque) tels que décrits ci-dessus, comprendrait les étapes suivantes :
- On dispose d'une prothèse et d'un instrument chirurgical (ou disque ou spirale) tels que décrits ci-dessus : dans une forme de réalisation de l'invention, le disque, dans sa configuration plate, est déjà logé au sein de la prothèse : dans une autre forme de réalisation, le chirurgien introduit le disque, sous sa forme plate, à l'intérieur de la prothèse : cette dernière est ainsi parfaitement déployée et légèrement rigidifiée par la feuille de matériau constituant le disque ;
- On pratique une incision de la paroi abdominale au niveau du défaut herniaire ;
- Après traitement du défaut herniaire, on introduit dans l'incision ladite prothèse, dans laquelle est logé le disque, en la pliant en exerçant une pression sur les bords du disque;
- Grâce à son effet ressort, le disque se déploie dans la cavité abdominale, réalisant l'étalement de la prothèse ; on positionne ladite prothèse contre la paroi abdominale, en la centrant sur le défaut par exemple en tirant sur les fils de centrage de la prothèse, la feuille de matériau du disque rigidifiant la prothèse et la plaquant contre la paroi abdominale de telle sorte qu'on évite que des viscères ne s'interposent entre ladite prothèse et la paroi abdominale ; un étalement correct de ladite prothèse est ainsi assuré ;
- On fixe la prothèse contre la paroi abdominale grâce à des moyens d'attache ;
- On tire ensuite sur la languette d'extraction située à l'extrémité supérieure de la spirale du disque ; ce faisant, on déploie la spirale et on extrait le disque en le faisant tourner lentement sur lui-même.

## Revendications

1. Kit chirurgical (10) pour le traitement d'un défaut herniaire de la paroi abdominale (101) comprenant :
- un instrument chirurgical comprenant au moins une feuille (2) en matériau souple et élastique, ladite feuille se superposant sur elle-même une ou plusieurs fois de façon continue afin de définir plusieurs niveaux (a, b, c) formant une spirale (3),
et une prothèse (200) destinée à combler ledit défaut, ladite prothèse comprenant au moins une première couche (201) en matériau souple biocompatible destinée à être placée en regard de la paroi abdominale et au moins une deuxième couche (202) en matériau souple biocompatible destinée à être placée en regard de la cavité abdominale (109), lesdites première et deuxième couches étant assemblées entre elles de façon à définir un espace interne (203) accessible audit instrument chirurgical (1) au moyen d'un orifice (204) ménagé dans la dite première couche.

2. Kit chirurgical (10) selon la revendication précédente, **caractérisé en ce que** ladite spirale (3) est apte à adopter une configuration substantiellement plate, dans laquelle chaque niveau (a, b, c) est en contact avec le niveau adjacent, correspondant à une configuration au repos dans laquelle la spirale (3) ne subit pas de contraintes significatives, ou au contraire une configuration déployée, dans laquelle aucun niveau (a, b, c) n'est en contact avec un autre niveau, correspondant à une configuration dans laquelle une force tendant à éloigner l'une de l'autre les deux extrémités (3a, 3b) de la spiracle (3) est exercée.

3. Kit chirurgical (10) selon la revendication 1 ou 2, **caractérisé en ce que** l'extrémité supérieure (3a) de la spirale (3) comprend une languette d'extraction (4).

4. Kit chirurgical (1) selon l'une quelconque des revendications 1 à 3, **caractérisé en ce que** le matériau constituant ladite feuille est un polymère choisi parmi le polypropylène, le polyéthylène, le polytétrafluoréthylène (PTFE) et/ou leurs mélanges.

5. Kit chirurgical (10) selon l'une quelconque des revendications 1 à 4, **caractérisé en ce qu'**il comporte une pluralité de fils de centrage (13) destinés à être liés à ladite prothèse sur le pourtour dudit orifice (204).

6. Kit chirurgical (10) selon l'une quelconque des revendications 1 à 5, **caractérisé en ce qu'**au moins une des couches (201 ; 202) de ladite prothèse est constituée d'un arrangement de fils.

7. Kit chirurgical (10) selon la revendication 6, **caractérisé en ce que** les deux couches (201, 202) sont constituées d'un arrangement de fils.

8. Kit chirurgical (10) selon l'une quelconque des revendications 1 à 7, **caractérisé en ce que** la deuxième couche (202) de ladite prothèse du kit est recouverte d'un revêtement anti-adhérent (206) sur sa face destinée à être mise en regard de la cavité abdominale (109).

## Claims

1. A surgical kit (10) for treating a hernia of the abdominal wall (101) comprising
- a surgical instrument (1) including at least one sheet (2) made of a flexible resilient material, said sheet continuously overlapping itself one or more times so as to define a plurality of levels (a, b, c) forming a spiral (3), and
- a prosthesis (200) intended to fill in said defect, said prosthesis including at least one first layer (201) made from a biocompatible flexible material intended to be placed opposite the abdominal wall, and at least one second layer (202) made from a biocompatible flexible material intended to be placed opposite the abdominal cavity (109), said first and second layers being assembled together so as to define an internal space (203) accessible to said surgical instrument (1) by means of an opening (204) provided in said first layer.

2. The surgical kit (10) according to the preceding claim, **characterized in that** said spiral (3) is able to adopt a substantially flat configuration, in which each level (a, b, c) is in contact with the adjacent level, corresponding to an idle configuration in which the spiral (3) does not undergo significant stresses, or on the contrary a deployed configuration, in which no level (a, b, c) is in contact with another level, corresponding to a configuration in which a force tending to space the two ends (3a, 3b) of the spiral (3) away from each other is exerted.

3. The surgical kit (10) according to claim 1 or 2, **characterized in that** the upper end (3a) of the spiral (3) comprises a removal tab (4).

4. The surgical kit (10) according to any one of claims 1 to 3, **characterized in that** the material making up said sheet is a polymer chosen from polypropylene, polyethylene, polytetrafluoroethylene (PTFE), and/or mixtures thereof.

5. The surgical kit (10) according to any one of claims 1 to 4, **characterized in that** it includes a plurality of centering threads (13) intended to be connected to said prosthesis around the perimeter of said opening (204).

6. The surgical kit (10) according to any one of claims 1 to 5, **characterized in that** at least one of the layers (201; 202) of said prosthesis is made up of an arrangement of threads.

7. The surgical kit (10) according to claim 6, **characterized in that** both layers (201, 202) are made up of an arrangement of threads.

8. The surgical kit (10) according to any one of claims 1 to 7, **characterized in that** the second layer (202) of said prosthesis of the kit is covered with an anti-adhesive coating (206) on its face intended to be placed opposite the abdominal cavity (109).

## Patentansprüche

1. Operationskit (10) zur Behandlung eines Bruchs der Bauchwand (101), das umfasst:
- ein Operationsinstrument, das mindestens einen Bogen (2) aus geschmeidigem und elastischem Material umfasst, wobei sich der Bogen kontinuierlich ein- oder mehrmals auf sich selbst legt, um mehrere Ebenen (a, b, c) zu definieren, die eine Spirale (3) bilden,
und eine Prothese (200), die dazu bestimmt ist, den Bruch auszufüllen, wobei die Prothese mindestens eine erste Schicht (201) aus biologisch verträglichem geschmeidigem Material umfasst, die dazu bestimmt ist, gegenüber der Bauchwand platziert zu sein und mindestens eine zweite Schicht (202) aus biologisch verträglichem geschmeidigen Material, die dazu bestimmt ist, gegenüber der Bauchhöhle (109) platziert zu sein, wobei die erste und die zweite Schicht derart miteinander verbunden sind, dass sie einen Innenraum (203) bilden, der für das Operationsinstrument (1) über eine Öffnung (204) zugänglich ist, die in die erste Schicht eingearbeitet ist.

2. Operationskit (10) nach vorangehendem Anspruch, **dadurch gekennzeichnet, dass** die Spirale (3) imstande ist, eine substantiell flache Konfiguration anzunehmen, in der jede Ebene (a, b, c) mit der benachbarten Ebene im Kontakt ist, die einer Ruhekonfiguration entspricht, in der die Spirale (3) keinen signifikanten Belastungen ausgesetzt ist, oder, im Gegenteil, eine entfaltete Konfiguration, in der keine Ebene (a, b, c) mit einer anderen Ebene im Kontakt ist, was einer Konfiguration entspricht, in der eine Kraft, die dazu neigt, die zwei Enden (3a, 3b) der Spirale (3) voneinander zu entfernen, ausgeübt wird.

3. Operationskit (10) nach Anspruch 1 oder 2, **dadurch gekennzeichnet, dass** das obere Ende (3a) der Spirale (3) eine Extraktionslasche (4) umfasst.

4. Operationskit (10) nach einem der Ansprüche 1 bis 3, **dadurch gekennzeichnet, dass** das Material, das den Bogen bildet, ein Polymer ist, das aus dem Polypropylen, dem Polyethylen, dem Polytetrafluorethylen (PTFE) und/oder ihren Gemischen ausgewählt ist.

5. Operationskit (10) nach einem der Ansprüche 1 bis 4, **dadurch gekennzeichnet, dass** es eine Vielzahl von Zentrierfäden (13) aufweist, die dazu bestimmt sind, mit der Prothese auf dem Umfang der Öffnung (204) verbunden zu sein.

6. Operationskit (10) nach einem der Ansprüche 1 bis 5, **dadurch gekennzeichnet, dass** mindestens eine der Schichten (201; 202) der Prothese von einem Fadenarrangement gebildet wird.

7. Operationskit (10) nach Anspruch 6, **dadurch gekennzeichnet, dass** die zwei Schichten (201; 202) von einem Fadenarrangement gebildet werden.

8. Operationskit (10) nach einem der Ansprüche 1 bis 7, **dadurch gekennzeichnet, dass** die zweite Schicht (202) der Prothese des Kits auf seiner Seite, die dazu bestimmt ist, gegenüber der Bauchhöhle (109) platziert zu sein, mit einer Antihaft-Beschichtung (206) beschichtet ist.
